# EUROPEAN PATENT APPLICATION

(11) **EP 0 922 456 A1**
(43) Date of publication of application: **16.06.1999**
(21) Application number: 97120699.0
(22) Date of filing: 26.11.1997
(51) Int. Cl.: A61K 31/215, A61K 31/14, A61K 31/205, A61L 15/20, A61F 13/15, A61K 7/48, A61K 31/22, A61K 31/225

(54) **Skin rash prevention composition**

(71) Applicant: THE PROCTER & GAMBLE COMPANY, Cincinnati, Ohio 45202 (US)
(72) Inventor: Palumbo, Gianfranco, 61348 Bad Homburg (DE); Guarracino, Mario, 64028 Silvi Marina (Teramo) (IT)
(74) Representative: Canonici, Jean-Jacques

(57) **Abstract**

The invention relates to ester compounds to be used for preparation of compositions, which can be applied to the skin to prevent or reduce or treat skin rash or diaper rash resulting from lipolytic dermatitis and which can be incorporated into lotions, creams, powders, foams, oils and the like. The compositions can also be applied to diapers, incontinent-pads, wipes and the like.

The invention also relates to the use of such a composition to prevent or reduce such a skin rash and a process for reducing the enzyme activity of the lipolytic enzymes being present on external skin, which are deactivated by theses compositions and/ or by lowering of the pH, on the external skin

## Description

The invention relates to ester compounds to be used for preparation of compositions, which can be applied to the skin to prevent or reduce or treat skin rash or diaper rash resulting from lipolytic dermatitis and which can be incorporated in lotions, creams, powders, foams, oils and the like. The compositions can also be applied to diapers, incontinent-pads, wipes and the like.

The invention also relates to the use of such a composition to prevent or reduce such a skin rash and a process for reducing the enzyme activity of those lipolytic enzymes being present on external skin which are deactivated by those compositions and / or by lowering of the pH.

### Background

Skin rash caused by dermatitis, often referred to as diaper rash, has always been a problem encountered by the users of disposable absorbent articles, such as diapers, incontinence articles, sanitary towels, training pants etc. Therefore, one of the biggest needs for these users is a solution to this type of skin rash problem.

The main factor which influences the development of skin rash is the contact of the skin with the wet body exudates, directly or for example contained in the absorbent article. Especially when the water content is high, skin rash can occur easily.

Manufacturers of diapers and skin care products have developed various products over the past decades which help reduce the occurrence of diaper rash (or skin rash).

The main focus thereby has been to reduce the exposure of the skin to the body exudates. This is for example done by introduction to the diaper of absorbing or better absorbing materials. The amount of water which is in contact with the skin is thus reduced.

Other products which are developed to address the skin-rash problem reduce the exposure of the skin to certain ingredients of the body exudates. An example of such ingredients of the exudate are bacteria which can infect the skin and thus start off or aggravate the skin rash.

For example, lotions have been developed which can form a barrier between the skin and the body exudates. Also, anti-inflammatory compositions can be applied to the skin or absorbent article.

EP 0191128 discloses a preparation comprising 8-hydroxy quinoline sulphate for treatment of skin irritation.

However, still one of the most heard complaints amongst users of absorbent articles such as diapers is the persistence of skin or diaper rash, despite the numerous products on the market which can be applied to prevent diaper or skin rash.

It has been discovered that yet another factor can set off or aggravate skin rash, namely the presence in the body exudate of various enzymes, especially lipase enzymes.

EP 0117632B relates to disposable articles which comprise lipase inhibiting agents, preferably zinc containing components, and a vehicle material.

US 3, 961,486 teaches the use of adipic acid to reduce the lipase enzyme activity and to reduce the skin rash.

When the skin is exposed to lipase enzymes, for example by lipase enzymes in the body exudate, the lipid-containing components of the skin can be affected by these enzymes, The protection or barrier function of the top layer of the skin (the Strateum Corneum) will thus be diminished. This can effect the hearth of the skin and/or facilitate the infection of the skin. This can thus lead to skin or diaper rash.

It has been found that specific ester compounds can function as lipase enzyme substrates, which, when acted upon by a hydrolyzing lipase enzyme, being esterase enzymes, will be hydrolyzed resulting in the release of free acids. The inventors have found that, firstly, the presence of these acids will lower the pH of the area where the esters where topically applied to. This will amount to inactivation of all or most enzymes present in this area, in the body exudates, such as the lipase enzymes, protease enzymes. Secondly, the lipase enzymes are 'de-activated', because rather than hydrolysing the esters of the skin, such as lipids, they hydrolyse the alternative substrate, the ester compounds of the invention.

The inventors have found that the use of these ester compounds very effectively reduces or helps to prevent or treat the diaper/skin rash, resulting from dermatitis caused by in particular lipolytic esterase enzymes.

Thus, a process for reduction of the enzyme activity of the lipolytic enzymes on the external skin, is thus provided and encompassed herein.

### Summary of the Invention

The invention relates to the use of one or more ester compounds of formulas: 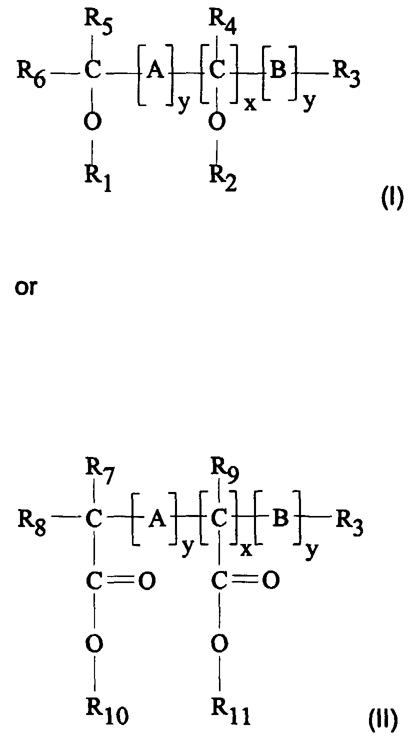 for preparation of a composition for prevention or treatment of lipolytic dermatitis wherein R₁ and each R₂ independently are an acyl group with from 2 to 22 carbon atoms, or an alkyl, alkenyl, arylalkyl, hydroxyalkyl group with from 1 to 24 carbon atoms or hydrogen, whereby at least one of R₁ and R₂ is such an acyl group, R₃ R₄, R₅, R₆, R₇, R₈, and R₉ are independently an alkyl, alkenyl, arylalkyl, hydroxyalkyl, alkoxy groups of from 1 to 24 carbon atoms, hydroxy group or hydrogen; R₁₀ and R₁₁ are independently an alkyl, alkenyl, arylalkyl, hydroxyalkyl, alkoxy groups of from 2 to 24 carbon atoms, hydroxy group or hydrogen; A and B are independently a C₁-C₆ linear or branched alkylene, alkenylene, alkoxylene, hydroxyalkylene groups; the values of x are independently from 0 to 15; the values of y are independently 0 or 1, with the proviso that when x =2 and y=0, at least one R₂ is an alkyl, alkenyl, arylalkyl, hydroxyalkyl group with from 1 to 24 carbon atoms or hydrogen

The invention also provides a disposable absorbent article, preferably a diaper, containing the compositions as described above, preferably such that the ester compounds therein are present at a level of from 0.01% to 10% by weight of the article, whereby it can be preferred that the composition is comprised in the topsheet of the diaper.

The invention also provides compositions, which are in the form of cosmetic compositions, in the form of a cream, lotion, gel, foam, oil, ointment or powder, which are preferably substantially free from calcium carbonate, for topical application to the external skin

### Detailed Description of the Invention

The ester compounds are used in the invention for the preparation of compositions for prevention, reduction or treatment of lipolytic dermatitis of the (external) skin.

These esters can function as enzyme substrates, which, when acted upon by a hydrolyzing lipolytic esterase enzyme, will be hydrolyzed resulting in the release of free acids. The presence of these acids will lower the pH of the area where the esters are topically applied to. This will amount to deactivation or inactivation of the lipolytic esterase enzymes present in this area, in the body exudates, which can otherwise affect the skin, resulting in irritation or skin rash.

By treatment or reduction is meant herein the reduction of the dermatitis or the rash of the skin which is caused by the lipolytic enzymes present on the skin, or at least stabilising the dermatitis or rash of the skin which is caused by these enzymes.

The composition prepared by use of the ester compounds of the invention can be applied to the skin which is in contact with at least the lipolytic esterase enzymes. Such composition can also be comprised in a cream, lotion, oil, ointment, foam, powder or gel, which can be topical applied to the skin.

Alternatively, the compositions of the invention can be applied to an absorbent article, which can be brought in close contact with the skin which is in contact with these lipolytic enzymes. Such articles are preferably disposable articles such as diapers, incontinent pads, training pants, sanitary towels, feminine hygiene garments, wet and dry wipes.

By the term "topical application" or "topical(ly) applied", as used herein, is meant directly laying on or spreading on epidermal tissue, especially outer skin.

The amount of the composition comprising the ester compounds of the invention will vary with the particular location of the condition being treated, the severity of the condition being treated, the expected duration of the treatment, any specific sensitivity to either the composition itself, or the concentration of the ester compounds specific to the user, the condition of the user, concurrent therapies being administered, other conditions present in the user.

For the present invention it is preferred that a minimum inhibitory concentration of the compositions containing the ester compound of the invention is topically applied, to act as lipolytic enzyme de-activator or inhibitor to the area in need of treatment of the lipolytic dermatitis or the area where prevention of lipolytictic dermatitis is desired in a form such that it is available to inhibit the activity of the lipase present.

This area (or "affected area", as used herein) is meant the area of the skin which is presently exhibiting any levels of skin rash or lypolitic dermatitis, or the area which will be in prolonged contact with body exudates containing the enzymes. This also includes the area immediately proximate to the described area. It is the area at which treatment, reduction of, and /or prevention is desired.

### Lipolytic dermatitis

This invention deals with compositions prepared by use of an ester compound as described above or (disposable) absorbent articles incorporating the compositions invention which can be used for the treatment of skin or in particular diaper rash or dermatitis caused by the lipolytic esterase enzymes present in the body exudates (thus lipolytic dermatitis) and other conditions which are associated with prolonged contact of the skin with body exudates and/or the wearing of a absorbent article, or in particular a diaper.

Lipolytic esterase enzymes are enzymes which can hydrolyse esters or compounds comprising an ester bond, such as lipids, thereby forming an acid or salt thereof and an alcohol.

Lipase, lipase enzyme or lipolytic enzyme is the trivial or common term employed to represent a group of enzymes belonging to the esterases. Their general activity is to hydrolyze fats present in the ester form (such as the glycerides found in human skin), and accordingly generate fatty acids and glycerol. Because this group of enzymes is so widely distributed in plants, moulds, bacteria, milk, and milk-products, as well as in almost all animal tissues, and because moreover human lipase enzymes are present in the pancreatic exudates, they are almost always present in body exudates.

The activity of enzymes, in particular lipase enzymes, contributes to almost all skin rash, in particular diaper rash, causing irritation by the digestive degenerative action of these enzymes on the skin per se and by breaking down the (lipid-) skin components, compromises the barrier property of the skin in the affected area. This breakdown of the integrity of the skin allows other components of the body exudates (urine and faeces in particular), which may not, by themselves, be irritating, to migrate through the compromised skin. At this point normally harmless components may then become irritating.

### Compositions

### Ester compounds

The present invention provides specific ester compounds, as defined above for use in the preparation of compositions which can be used for treatment, prevention or reduction of the skin rash or particularly diaper rash, which is set off or aggravated by lipolytic enzymes, thus being the result of lipolytic dermatitis.

It should be understood that for the purpose of this invention, the groups R₁-R₁₁ as defined above in formulations (I) and (II) can be branched or linear, and they can be substituted by any appropriate substituent group.

It can be preferred that the ester compound is a mono- or diester of formulation (I) or (II) above, having one or two acidic groups.

The presence of an acidic group in the ester compounds provides an acidity source, which can reduce the pH of the affected area, thereby aiding the inhibition or inactivation of the enzymes present in the body exudates on the skin.

Preferred are the ester compounds as defined above in formulation (I) and (II), wherein the compound is of formula I or II wherein x is 1 or more preferably 2 and y is 0.

A preferred ester compound has R₁ and one R₂ being a C₂-C₁₆ acyl group,.

Also preferred can be that R₁₀ or R₁₁, but most preferably R₁₀ and one or more R₁₁ is a C₁-C₁₆ alkyl group.

Highly preferred is that one or more of R₃ R₄, R₅, R₆, R₇ and R₈, or preferably all of R₃, R₄, R₅, R₆, R₇ and R₈, are methyl or more preferred hydrogen.

It can be highly preferred that the ester compound is a glycerol mono- or di-acetate, or a mono- or diester or triester of citric acid, or a mono- or diester of tartaric acid, such as triethyl citrate, diisopropyl tartrate, diethyl tartrate, dibuthyl L-tartrate,

The composition preferably comprise the ester compounds at a level of from 0.01% to 90%, more preferably from 0.5% to 60%, most preferably from 2% to 25% by weight of the composition.

The compositions can be prepared by any conventional formulation technique known in the art.

### Lotions, creams, oils, foams, ointments, gels, powders and the like

The compositions prepared by the use of the ester compounds according to the invention can also be in the form of cosmetic compositions, in the form of lotions, creams, oils, ointments, powders, foams, gels which can comprise any of the ingredients commonly used in the art for such compositions.

When the cosmetic compositions of the invention are for application on the skin, they are preferably in such a form that they can be applied to the skin as a leave-on product.

The cosmetic cream, lotion, gel, oil, ointment or powder are preferably substantially free from carbonate salts such as calcium carbonate.

It is to be understood that the ingredients of the compositions above will depend on the character of the composition, thus lotions will generally comprise different additional ingredients than powders.

In the cosmetic creams, lotions, gels, oils or powders comprising the composition of the invention preferably an acidity source is present, preferably such that is capable to reduce the pH of the skin to below a pH of 8, more preferably below a pH of 7, more preferably below a pH of 6, or it can even be more preferred to be below a pH of 5.

A wide variety of optional ingredients such as non-occlusive moisturisers, humectants,
gelling agents, neutralising agents, perfumes, colouring agents, can be added to the skin compositions herein.

Other additional ingredient can be anionic, nonionic, cationic, amphoteric and amphiphilic surfactants, which are known in the art.

### Absorbent Articles

The compositions of the present invention can be comprised in a absorbent article, preferably a disposable absorbent article. A particularly preferred absorbent articles therefor is a diaper, which preferably comprises the composition in the topsheet of the diaper.

As used herein, the term "absorbent articles" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner).

The structure of the disposable absorbent article is not critical to the practice of the present invention.

Normally, the composition is incorporated into the absorbent article or diaper in particular in an amount which will deliver the required treatment or reduction or prevention of the lipolytic dermatitis preferably after frequent use.

The disposable absorbent article preferably contains the composition according to the invention preferably at such a level that that the ester compounds therein are present at a level of from 0.01% to 30%, more preferably from 0.01% to 10%, most preferably from 0.05% to 5% by weight of the article.

An absorbent article generally comprises
- an absorbent core (which may consist of sub-structures);
- a fluid pervious topsheet;
- a fluid impervious backsheet;
- optionally further features like closure elements or elastification.

As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments, and the like.

A preferred wipe for the purpose of this invention comprises an absorbent fibrous material or core into which the composition may be releasably incorporated. A highly preferred disposable wipe for the purposes of this invention comprises an absorbent fibrous material and a faeces-impermeable backing material; said backing being superposed or co-extensive with one face of said absorbent fibrous material; said backing material most preferably being a web-backing material and most preferably having a width greater than said absorbent material providing side marginal portions which extend beyond said absorbent material, said margin portions being folded around and on top of the edges of said absorbent material. The compositions of the invention agent may be releasably incorporated into the wipe structure by diverse methods which will be readily apparent to those skilled in the art. For example, the compositions can be present in aqueous or volatile carrier such as water, ethanol, or the like, or creams. lotions, oils, ointments, gels or powders, and applied to the absorbent material by spraying, dipping, printing, soaking or otherwise contacting the absorbent material of the wipe with the lipase-inhibiting agent and its carrier. A skin cleansing agent, preferably an oleaginous cleansing agent, may optionally be releasably incorporated into the absorbent material as well.

The compositions of the present invention are preferably incorporated into a diaper, preferably into the absorbent core structure or most preferably into the topsheet structure. The composition may be incorporated into the diaper structure by diverse methods which will be readily apparent to those skilled in the art. For example, the composition can be, optionally after being dispersed aqueous or volatile carrier such as water, ethanol, or the like, applied to the diaper topsheet, to the absorbent core, or to the core side of the backsheet, by spraying, dipping, printing, soaking or otherwise contacting the selected structural element of the diaper with composition and optionally its carrier, which is called herein impregnation.

The diaper preferably comprises a liquid pervious topsheet, a liquid impervious backsheet joined with the topsheet, an absorbent core positioned between the topsheet and the backsheet. While the topsheet, the backsheet, and the absorbent core may be assembled in a variety of well known configurations, preferred diaper configurations are described generally in U.S. Patent 3,860,003 entitled "Contractable Side Portions for Disposable Diaper" which issued to Kenneth B. Buell on January 14, 1975; and U.S. Patent Application Serial No. 07/715,152, allowed, "Absorbent Article With Dynamic Elastic Waist Feature Having A Predisposed Resilient Flexural Hinge", Kenneth B. Buell et al. filed June 13, 1991.

The backsheet is positioned adjacent the garment surface of the absorbent core and is preferably joined thereto by attachment means such as those well known in the art. For example, the backsheet may be secured to the absorbent core by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The absorbent article may further comprise elastification or closure features well-known in the art and - for example - described in E 0254476 (Alemany).

The topsheet is positioned adjacent the body surface of the absorbent core and is preferably joined thereto and to the backsheet by attachment means such as those well known in the art. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

Generally, the topsheet is compliant, soft feeling, and non-irritating to the wearer's skin. Further, the topsheet is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques which may be used to manufacture the topsheet. For example, the topsheet may be a nonwoven web of fibres spunbonded, carded, wet-laid, meltblown, hydroentangled, combinations of the above, or the like.

Preferably the topsheet comprises a means to adjust hydrophilicity of the material.

Absorbent cores comprise essentially all absorbent parts of the absorbent article other than the topsheet, which contribute to fluid absorbency or fluid handling.

The absorbent cores should be generally compressible, conformable, non-irritating to the wearer's skin, and capable of absorbing and retaining liquids such as urine and other certain body exudates.

The absorbent core can be made of a variety of materials. Preferred materials are fibrous materials, which can form a fibrous web, natural occuring or synthetic fibres or optionally thermoplastic fibres. In addition thereto polymeric stiffening agents are preferably present. Also preferred can be hydrogel-forming polymers or absorbent polymers.

The various core, topsheet and backsheet materials can be arranged in any way known in the art, such as described in Weisman et al. (EP 0 202 125) or Alemany et al. (EP 0 254 476).

Also encompassed in the present invention is a process for making a diaper comprising the composition of the invention whereby the topsheet is impregnated with the composition before incorporation in the diaper..

### Additional ingredients

The composition of the invention can comprise additional ingredients. Which ingredient are present and in which level depends on the character of the composition and the use thereof.

Another highly preferred ester compound for use in the preparation of the compositions of the present invention or for use in the compositions can be an ester compound of the formulation: 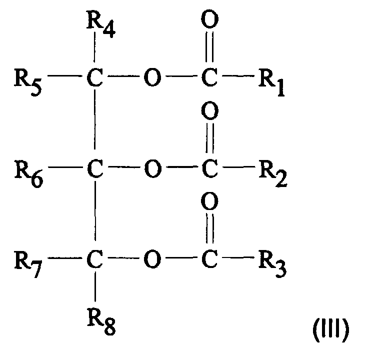 wherein R₁, R₂ and R₃ are independently an alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, alkenyl or hydroxyalkyl groups, hydroxy, chloride, bromide, amine or hydrogen.

Highly preferred are the compounds above wherein R₄, R₅, R₆, R₇ and R₈ of said compound are hydrogen and preferably wherein R₁, R₂ and R₃ are independently an C1-C4 alkyl group.

Such a preferred compound can be glycerol triacetate.

It has been found that another highly preferred additional component to be used for the preparation of the compositions of the present invention or for use in the compositions of the present invention are certain cationic compounds.

The lipase has been found to be activated by the presence of pancreatic bile salts, which are mostly present in the body exudates. The bile salts function as emulsifiers, enabling the lipase enzymes to act on the water-lipid interface.

When employing cationic compounds in the compositions of the present invention, the bile salts are inactivated and thus the lipase is inactivated. It is thereby prevented form acting upon the skin and causing irritation. Such inactivation of lipase prevents the compromise of the barrier function of the skin which in turn prevents irritants (such as fungi, bacteria, and bile salts and acids) form migrating through and further irritating and inflaming the skin.

In the compositions of the present invention the use of such a cationic compound has been found to have a surprising effect: it has been found that the cationic compounds provide an immediate effect, i.e. immediate inhibition or inactivation of the lipase enzymes, which may reduce over time, whilst the effect of the ester compounds is relatively delayed but long-lasting. Thus, the combination of the two compounds provides an very effective, immediate and long-lasting reduction or prevention of the diaper/skin rash, resulting from dermatitis caused by the enzymes present on the body exudates on the skin.

The cationic compounds for use in the compositions of the invention are preferably of the of formulations: 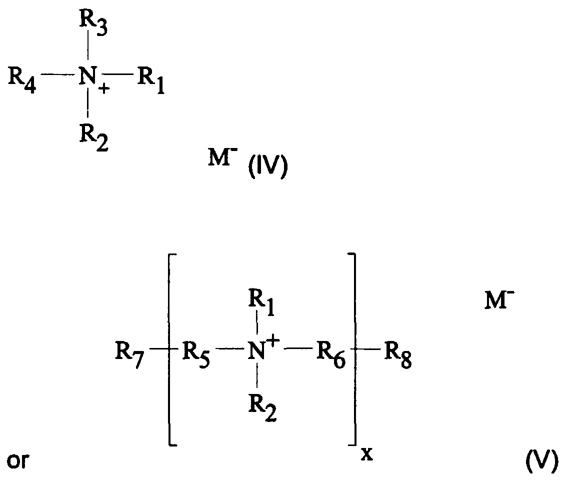 or an amphoteric compound and preferably an acidity source, the amphoteric compound having at its iso-electric point the formula: 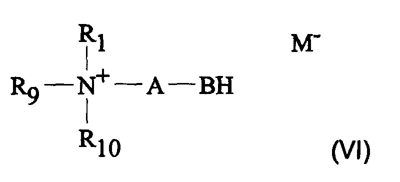 wherein R₁, R₂, R₃ and R₄ are independently a C₁-C₂₂ alkyl, alkenyl, aryl, arylalkyl, amidoalkyl, (poly) alkoxy, hydroxyalkyl, or acyl groups, or two or more groups of R₁, R₂, R₃ and R₄ form together one or more ring structures; R₅, R₆ and A are independently a C₁-C₂₂ alkylene, alkenylene, (poly) alkoxylene, hydroxyalkylene, arylalkylene or amido alkylene groups; R₇ and R₈ are independently a C₁-C₄ alkyl, alkenyl, alkoxy group or a hydroxy group or hydrogen; R₉ and R₁₀ are independently a C₁-C₂₂ linear or branched alkyl, alkenyl, aryl, arylalkyl, amidoalkyl, (poly) alkoxy, hydroxyalkyl, or acyl groups, or two or more of the groups R₁, R₉ and R₁₀ form together one or more ring structures; BH is a proton donating group; x is from 2 to 4; and M- is a counter ion.

It should be understood that for the purpose of this invention, the groups R₁-R₁₀ of formulations (IV), (V) and (VI) above can be substituted by any appropriate substituent group.

In the formulations (IV), (V) and (VI) above, R₁, R₂, R₃ and R₉ are independently preferably C₁-C₈, more preferably C₁-C₄ alkenyl or alkoxy, more preferably alkyl groups, most preferably methyl or ethyl groups.

Preferably, R₄, R₅ and R₁₀ are independently C₈-C₁₈, more preferably C₁₂-C₁₆ alkenyl or alkoxy, more preferably alkyl or arylalkyl groups, whereby it can be preferred that one of the R₄, R₅ and R₉ substituents is benzyl group.

Alternatively, it can be preferred that the cationic compound comprises at
least
one R₁, R₂ or R₃ or R₉ being a poly alkoxy group.

Thus, R₁, R₂, R₃ and R₉ preferably are independently polyalkoxy groups comprising C₂-C₆, preferably C₂-C₃ alkoxy units and having an alkoxylation number of from 2 to 50, preferably from 5 to 18. Then, R₃, R₄ and R₁₀ are independently preferably C₁-C₈, more preferably C₁-C₄ alkenyl or alkoxy, more preferably alkyl groups, most preferably methyl or ethyl groups.

A, R₅ and R₆ are, independently, preferably C₁-C₆ alkenylene or more preferably alkylene groups, most preferably methylene or ethylene.

Preferred compounds can be benzalkonium chloride or Merquat 2200 (Trade name, being a 2-Propeneamide polymer of N,N-dimethyl-N-2-Propenyl-1-amonium chloride).

Preferred cationic compounds of the formulas above comprise one or more substituted R₁, R₂, R_{3,}R_{4,} R₉ or R₁₀ groups and/ or a substituted R₅ and/or substituted R₆ group, whereby the substituent is selected from the group from the group consisting of derivatives of silicon, glucose, fructose and saccharose.
Preferred can be Glucquat 125 (trade name, being lauryl dimethyl glucet-10-hydroxydimonium chloride).

Preferred cationic compounds of the formula (VI) above are betaine or sulpho betaine
having preferably R₁ and R₉ being a methyl group.

The additional cationic compounds or additional triester compounds are preferably present in the compositions of the invention at a level of from 0.01% to 20%, more preferably from 0.05% to 10%, most preferably from 0.1% to 5% by weight of the composition.

### Process

Also encompassed in the invention is a process for reducing the enzyme activity of the lipolytic esterase enzymes present on the external skin, whereby the process comprises the steps of preferably topical, applying of a composition of the invention to the external skin, or preferably topical, applying of a composition according to the invention to the external skin.

Thereby, acids are formed (in situ), preferably capable of reducing the initial pH to below 7.9, more preferably below 7.5 or even 7.3.

It can be preferred that an additional acidity source is present, capable of reducing the pH to below 7.3, preferably below 6 or even 5.

The composition used in the process or the process is preferably such that within the first 15 minutes after application of the composition to the affected area, the lipase enzyme activity is reduced to 35%, preferably 25%, more preferably less than 20% of the initial lipase activity in this area.

Preferably the composition used in the process or the process is such that 60 minutes after application of the composition to the affected area, the lipase enzyme activity is still less than 45%, preferably 35%, more preferably less than 30% of the initial lipase activity in this area.

This process can be preferably done by applying an absorbent article which comprises the composition present in a suitable level, to the skin.

## Claims

1. The use of an ester compound of the formulation: 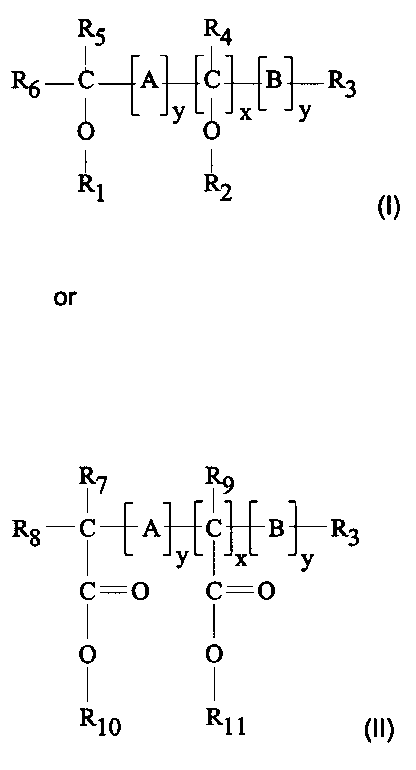 for preparation of a composition for prevention or treatment of lipolytic dermatitis wherein R₁ and each R₂ independently are an acyl group with from 2 to 22 carbon atoms, or an alkyl, alkenyl, arylalkyl, hydroxyalkyl group with from 1 to 24 carbon atoms or hydrogen, whereby at least one of R₁ and R₂ is such an acyl group, R₃ R₄, R₅, R₆, R₇, R₈, and R₉ are independently an alkyl, alkenyl, arylalkyl, hydroxyalkyl, alkoxy groups of from 1 to 24 carbon atoms, hydroxy group or hydrogen; R₁₀ and R₁₁ are independently an alkyl, alkenyl, arylalkyl, hydroxyalkyl, alkoxy groups of from 2 to 24 carbon atoms, hydroxy group or hydrogen; A and B are independently a C₁-C₆ linear or branched alkylene, alkenylene, alkoxylene, hydroxyalkylene groups; the values of x are independently from 0 to 15; the values of y are independently 0 or 1, with the proviso that when x=2 and y=0, at least one R₂ is an alkyl, alkenyl, arylalkyl, hydroxyalkyl group with from 1 to 24 carbon atoms or hydrogen

2. The use of an ester compound for preparation of a composition according to Claim 1 wherein the composition is for prevention or treatment of diaper rash.

3. The use of an ester compound for preparation of a composition according to Claim 1 or 2 wherein the compound is of formula I or II wherein x=2, y=0; R₁, and one R₂ are C₂-C₁₆ acyl groups, R₁₀ and one or more R₁₁ are a C₁-C₁₆ alkyl group; R₃, R₄, R₅, R₆, R₇, R₈, and R₉ are hydrogen.

4. The use of a compound for preparation of a composition according to any of Claims 1 to 3 wherein the compound is a monoester or diester of citric acid or the salt thereof or a triester of citric acid or a monoester of tataric acid or a salt thereof or a diester of tartaric acid.

5. The use of a compound for preparation of a composition according to any of Claims 1 to 3, wherein the compound is glycerol mono- or di-acetate.

6. A composition according to any preceding Claim, whereby the ester compound is present at a level of from 0.01% to 90% by weight.

7. The use of a compound for preparation of a composition according to any proceeding Claim, whereby additionally is used a cationic compound of the formulation: 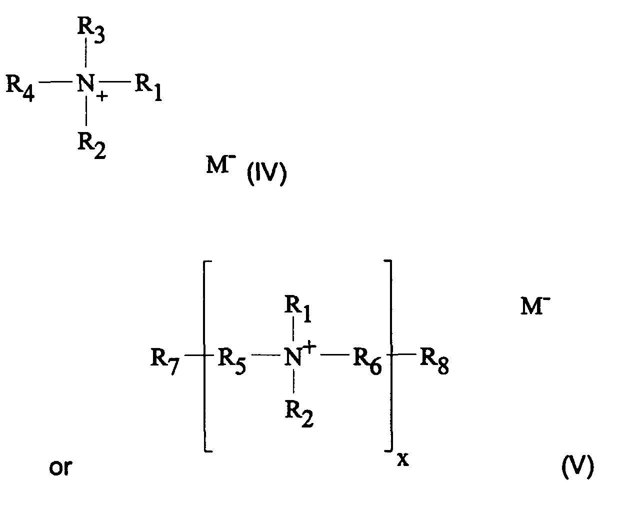 or an acidity source and an amphoteric compound having at its iso-electric point the formula: 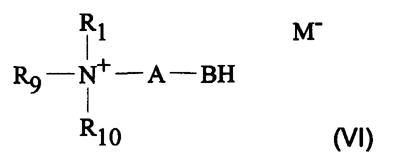 wherein R₁, R₂, R₃ and R₄ are independently selected from the group consisting of C₁-C₂₂ linear or branched alkyl, alkenyl, aryl, arylalkyl, amidoalkyl, (poly) alkoxy, hydroxyalkyl, or acyl groups, or two or more groups of R₁, R₂, R₃ and R₄ form together one or more ring structures; R₅, R₆ and A are independently selected from the group consisting of C₁-C₂₂ linear or branched alkylene, alkenylene, (poly) alkoxylene, hydroxyalkylene, arylalkylene or amido alkylene groups; R₇ and R₈ are independently an C₁-C₄ alkyl, alkenyl, alkoxy group or a hydroxy group or hydrogen; R₉ and R₁₀ are independently selected from the group consisting of C₁-C₂₂ linear or branched alkyl, alkenyl, aryl, arylalkyl, amidoalkyl, (poly) alkoxy, hydroxyalkyl, or acyl groups, or two or more of the groups R₁, R₉ and R₁₀ form together one or more ring structures; BH is a proton donating group; x is from 1 to 4; and M-is a counter ion.

8. The use of an ester compound for preparation of a composition according to any preceding Claims, whereby the composition comprises a cationic compound according to Claim 7.

9. The use according to Claim 7 or 8 wherein the cationic compound comprises one or more substituted R₁, R₂, R₃, R₄, R₉ or R₁₀ groups and/ or a substituted R₅ and/or substituted R₆ group, whereby the substituent is selected from the group from the group consisting of derivatives of silicon, glucose, fructose and saccharose.

10. The use according to Claim 7 or 8 wherein the cationic compound comprises at least one R₁, R₂, R₃ and R₉ being C₁-C₈, preferably C₁-C₄ alkyl, alkenyl or (poly) alkoxy groups, most preferably methyl or ethyl groups.

11. The use according to Claim 7 or 8 , wherein the cationic compound is of formula (VI), being a betaine compound or sulphobetaine compound.

12. A composition according to Claim 6, whereby the cationic compound according to any of Claims 6 to 10 is present at a level of from 0.01% to 20% by weight of the composition.

13. The use of a compound for preparation of a composition according to any proceeding claim, whereby an additional ester compound of the formulation is used: 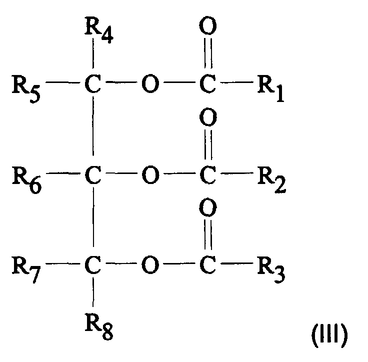 wherein R₁, R₂ and R₃ are independently an alkyl or alkenyl or hydroxyalkyl group with from 1 to 22 carbon atoms, and R₄, R₅, R₆, R₇ and R₈ are independently selected from the group consisting of C₁ - C₁₀ linear or branched alkyl, alkenyl or hydroxyalkyl groups, hydroxy, chloride, bromide, amine or hydrogen.

14. The use of a compound for preparation of a composition according to any proceeding claim, whereby the additional ester compound according to Claim 13 is glycerol triacetate.

15. A disposable absorbent article containing a composition containing the ester compound according to any of Claims 1 to 5 according to any proceeding Claim, the ester compound being present at a level of from 0.01% to 10% by weight of the article.

16. A disposable absorbent article according to Claim 15, in the form of a diaper whereby the diaper comprises a topsheet, containing the compound according to any preceding Claim.

17. A process for making a diaper according to Claim 16, whereby the topsheet is impregnated with the composition according to any preceding Claim prior to incorporation of the topsheet in the diaper.

18. A cosmetic composition according to any of Claims 1 to 14 in the form of a foam cream, lotion, gel, oil, ointment or powder, for topical application to the external skin or to a disposable absorbent article, which is substantially free from carbonate salts.

19. A process for reducing the enzyme activity of the enzymes present on the external skin comprising the steps of (topically) applying a composition according to any of Claims 1 to 5.

20. A process for reducing the enzyme activity of the enzymes present on the external skin comprising the steps of (topically) applying a composition or absorbent article comprising the composition according to any preceding Claim to the external skin.
